(19) Europäisches Patentamt
European Patent Office
Office européen des brevets

(11) Publication number: 0 094 738
B1

(12) EUROPEAN PATENT SPECIFICATION

(45) Date of publication of patent specification: 26.11.86

(51) Int. Cl.$^4$: C 07 D 319/08, A 61 K 31/335

(21) Application number: 83301673.6

(22) Date of filing: 24.03.83

# (54) Pharmaceutical benzodioxane compounds and process for their manufacture.

(30) Priority: 24.03.82 AU 3285/82

(43) Date of publication of application:
23.11.83 Bulletin 83/47

(45) Publication of the grant of the patent:
26.11.86 Bulletin 86/48

(84) Designated Contracting States:
AT BE CH DE FR GB IT LI NL SE

(58) References cited:
GB-A-1 539 149

(73) Proprietor: R.P. SCHERER CORPORATION
2075 West Big Beaver Road
Troy Michigan 48099 (US)

(72) Inventor: Bateman, Neil E.
23 Edro Avenue East Brighton
Melbourne Victoria (AU)
Inventor: Woods, Ross A.
Flat 11, 10 Murry Street
West Brunswick Victoria 3055 (AU)

(74) Representative: Allam, Peter Clerk et al
LLOYD WISE, TREGEAR & CO. Norman House
105-109 Strand
London WC2R 0AE (GB)

EP 0 094 738 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to new pharmaceutical compounds, to a process for the preparation thereof, and to pharmaceutical compositions incorporating the new compounds. In particular, the present invention relates to analgesic and anti-inflammatory compounds.

Acetyl salicylic acid, or aspirin as it is more commonly known, is amongst the most widely used of proprietary medicines. Asparin can be used in the treatment of numerous ailments and is indicated to have analgesic, anti-inflammatory, antipyretic and antirheumatic activity. However, side effects of the drug may limit its application. For example, acetyl salicylic acid may cause gastric irritation and is contra-indicated where such irritation must necessarily be avoided.

GB—A—1539149 discloses that acetyl salicylic acid derivatives of the formula A below possess analgesic and anti-inflammatory activity without causing gastric irritation:

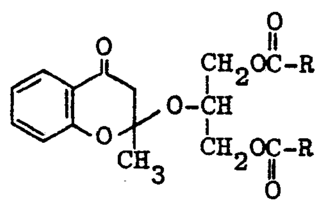

(A)

wherein each R represents an alkyl group containing from 1 to 15 carbon atoms. However, this prior art document stresses the importance of the glyceride moiety, and establishes that a structurally similar compound, 2 - hexadecyloxy - 2 - methyl - 1,3 - benzodioxan - 4 - one, does not possess the same advantageous therapeutic properties when administered to rats.

The object of the present invention is to provide pharmaceutical substances and compositions which exhibit some or all of the advantageous properties of aspirin but in which the contra-indications are minimized.

According to the present invention, it has been discovered that certain new esters of acetyl salicylic acid derivatives provide useful pharmaceutical activity.

More specifically, the present invention provides a compound of the formula I:

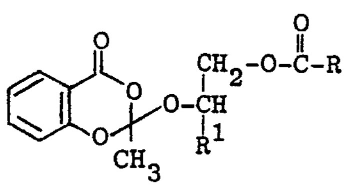

I

In formula I, R and $R^1$, which may be the same or different, represent an alkyl group containing from 1 to 15 carbon atoms.

The present invention also includes pharmaceutically acceptable acid addition salts of the compounds of formula I.

In a preferred form R represents a straight chain alkyl group. In a more preferred form R represents a straight chain alkyl group of from 7 to 11 carbon atoms. In a preferred form of the invention $R^1$ represents a lower alkyl group, for example, a methyl, ethyl or propyl group. A methyl group has been found to be most satisfactory.

Thus by way of example, a preferred class of compounds of the present invention are of the formula II:

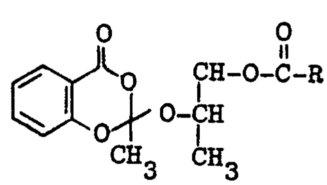

II

where R is a straight chain alkyl radical having from 1 to 15 carbon atoms and preferably from 7 to 11 carbon atoms.

The compounds of the present invention have analgesic and anti-inflammatory activity similar to those of acetyl salicylic acid. It has been found that the following tests provided a valuable guide to the activity of the compounds of the present invention. These tests are the animal model experiments described by L. B. Witkin *et al., J. Pharmacol. Exptl. Therap., 133,* 400 (1961) and C.A. Winter *et al., Proc Soc., Exp. Biol. Med., III,* 544 (1962). The use of compounds of the formula I is particularly advantageous since gastric irritation associated with acetyl salicylic acid therapy is avoided or at least minimized.

It is to be understood that this invention encompasses the individual isomeric forms of the compounds

as well as mixtures thereof. This includes optically active isomers and racemic mixtures thereof. The benzodioxane derivatives of the present invention may be manufactured by any chemical process known to be useful for the manufacture of chemically analogous compounds.

Compounds of the general formula I may be prepared as a mixture of diasteriomers. Such diasteriomers are separable by standard techniques e.g. chromatography.

A preferred process for the manufacture of a benzodioxane derivative of the present invention comprises the reaction of an acetyl salicylic acid derivative with a monoglycol ester of the general formula III:

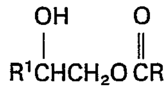

III

wherein R and $R^1$ have the meanings given above. Standard techniques may be utilized in the reaction between acetyl salicylic acid and the monoglycol esters of formula III for the preparation of 2-alkoxy-4-oxo-1, 3 benzodioxandes as exemplified by C. Ruchardt and S. Rochlitz. *Liebigs Ann. Chem.,* 15 (1974) and G. Y. Paris *et al., J. Med. Chem. 23* 79 (1980). For example, the acetyl salicylic acid may be activated by conversion to its acylhalide or mixed anhydride to form a 1, 3-benzodioxan-4-one. 1,3-benzodioxan-4-one may be subsequently reacted with monoglycol ester of formula III to produce the compounds of the general formula I. The halide may be selected from a chloride, bromide and iodide. An acid chloride is preferred.

Compounds of the formula III may be prepared via an acylation reaction of a hydroxyketone of the formula IV:

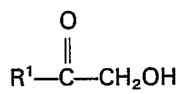

IV

wherein $R^1$ has the meaning given above; with a carboxylic acid of the formula V:

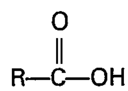

V

wherein R has the meaning given above; to yield compounds of the formula VI:

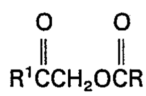

VI

A particularly preferred hydroxyketone is the monohydroxy acetone. This yields a monoglycol ester of the formula VII:

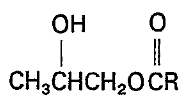

VII

This subsequently yields a benzodioxane derivative of the formula II:

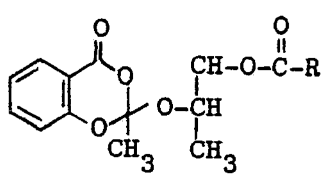

II

Particularly preferred compounds of the present invention which exhibit analgesic and anti-inflammatory activity but with reduced gastric irritation include 2 - (1 - Decanoyloxyprop - 2 - oxy) - 2 - methyl - 4 - oxo - 1, 3 - benzodioxane, 2 - (1 - Decanoyloxypropyl - 2 - oxy) - 2 - methyl - 4 - oxo - 1, benzodioxane, 1 - Octanoylpropan - 2 - ol and 2 - (1 - Octanoyloxyprop - 2 - oxy) - 2 - methyl - 4 - oxo - 1,3 - benzodioxane.

A suitable acid-addition salt of benzodioxane derivative of the invention is, for example, a salt derived from an organic acid, for example, a hydrochloride, hydrobromide, phosphate or sulphate, or a salt derived from an organic acid, for example an oxalate, lactate, succinate, tartrate, acetate, salicylate, citrate, benzoate, B-naphthoate, or adipate.

The benzodioxane derivatives of the present invention may be administered to animals including man, in the form of a pharmaceutical composition comprising as active ingredient at least one benzodioxane derivative of the present invention, or an acid-addition salt thereof, in association with a pharmaceutically

acceptable diluent or carrier therefor. The composition may further comprise a physiologically acceptable excipient, binder, preservative, stabilizer, flavoring or other compounding ingredient. The composition may be prepared in a conventional unit dosage form as called for by accepted pharmaceutical practice. The composition may be included in soft gelatin capsules, two piece hard gelatin shell capsules, tablets, elixirs, suspensions, emulsions or injectible solutions or suspensions or oily solutions or suspensers.

The amount of active substances include is selected so as to provide an individual unit dosage, preferably from about 30 milligrams to 1500 milligrams of the active ingredient. Other therapeutically valuable substances may also be included. Caffeine, phenacetin, paracetamol may be used in addition to the active ingredient of the present invention.

The following examples are illustrative of the invention, or of intermediates which may lead to the invention, and represent preferred embodiments but are not to be construed as being limitations thereon. All temperatures are in degrees Celsius.

Example 1

1-Decanoyloxypropan-2-one

Monohydroxyacetone was distilled prior to use. Chloroform was distilled from phosphorous pentoxide prior to use. Decanoyl chloride (65 $cm^3$, 0.3146 mole) was added dropwise to stirred solution of monohydroxyacetone (21.8 $cm^3$, 0.3146 mole) and anhydrous pyridine (25.4 $cm^3$, 0.3146 mole) in anhydrous chloroform (500 $cm^3$). During the addition the solution was maintained in an ice bath. The mixture was then stirred at room temperature overnight. The solution was washed with water (4 × 200 $cm^3$) and saturated sodium chloride solution (200 $cm^3$). The organic phase was dried over sodium sulphate and evaporated *in vacuo.* The resulting oil was distilled to yield 1-decanoyloxypropan-2-one as a mobile liquid, bp 154—60° (1 mm) (52.4 g, 0.230 mole, 73%), ir (neat) 2860, 1700, 1400, 1340, 1150, 1110 and 1050 $cm^{-1}$; pmr ($CDCl_3$) 4.6 (s, 2 H), 2.4 (t, 2 H), 2.1 (s, 3 H), 1.8—1.0 (bm, 14 H) and 0.9 (t, 3 H) ppm.

Example 2

1-Decanoyloxypropan-2-ol

1-Decanoyloxypropan-2-one (44.3 g, 0.194 mole) was dissolved in a solution of tetrahydrofuran (1100 $cm^3$) and benzene (200 $cm^3$). After cooling to 5°, ice water (80 $cm^3$) was added. Sodium borohydride (11 g, 0.291 mole) was added to the stirred solution in small proportions to maintain the temperature at 5°. After addition the reaction was stirred at 5° for 45 minutes and glacial acetic acid (14 $cm^3$) was added dropwise. Stirring at 5° was continued for a further 30 minutes. Diethyl ether and chloroform (200 $cm^3$ each) were added and the mixture washed with water (2 × 200 $cm^3$), a 1% sodium bicarbonate solution (200 $cm^3$) and brine (200 $cm^3$). The organic phase was dried over sodium sulphate and evaporated to yield 1-decanoyloxypropan-2-ol as a colorless liquid (44.0 g, 0.191 mole, 98%), ir(neat) 3600—3100, 2920, 2860, 1730, 1460, 1375, 1240, 1175, 1110, 1055 $cm^{-1}$; pmr ($CDCl_3$) 4.0 (m, 2 H), 3.6 (m, 2 HO ($D_2O$ m, 1 H), 2.3 (t, 2 H), 2.0—1.0 (btm. 17 H) and 0.85 (t, 3 H) ppm.

Example 3

2-(1-Decanoyloxyprop-2-oxy)-2-methyl-4-oxo-1, 3-benzodioxane

Trifluoroacetic anhydride (3.73 $cm^3$, 0.0263 $cm^3$) was added to a suspension of O-acetylsalicylic acid (4.3 g 0.239 mole) in anhydrous toluene (100 $cm^3$) at 80°. The solution was stirred at room temperature for 10 minutes. 1-Decanoyloxypropan-2-ol (5 g, 0.0217 mole) in toluene (25 $cm^3$) was added with rapid stirring. The solution was stirred at room temperature for 5 minutes and in ice for 10 minutes. Pyridine (7 $cm^3$, 0.0865 mole) was then added. Additional toluene (100 $cm^3$) was added and the solution extracted with water (2 × 150 $cm^3$), a saturated sodium bicarbonate solution (3 × 150 $cm^3$), dried over sodium sulphate and evaporated. The product was chromatographed on silica gel (600 g) in petroleum ether:ether (80:20) and treated with charcoal in acetone to yield 2 - (1 - decanoyloxyprop - 2 - oxy) - 2 - methyl - 4 - oxo - 1, 3 - benzodioxane as a mixture of diasteriomers (6.3 g, 0.0161 mole, 67%); ir (neat) 2920, 2850, 1730, 1600, 1580, 1470, 1380, 1310, 1275, 1250, 1150, 1080, 1015, 920 and 760 $cm^{-1}$; pmr ($CDCl_3$) 7.9 (dd, 2 H), 7.6—6.8 (m, 3 H), 4.6—3.6 (m, 3H) 2.2 (m, 2 H), 1.85 (s, 3 H), 1.6—1.0 (bm, 17 H) and 0.8 (m, 3 H) ppm.

| | | |
|---|---|---|
| Anal. calcd. for $C_{22}H_{32}O_6$: | C, 67.32; | H, 8.22% |
| Found: | C, 67.64; | H, 8.62% |

Example 4

2-(1-Decanoyloxypropyl-2-oxy)-2-methyl-4-oxo-1, 3-benzodioxane

A mixture of 1 equivalent of O-acetylsalicyloyl chloride, 1-decanoyloxypropan-2-ol and anhydrous pyridine in anhydrous, ethanol free, chloroform was heated at reflux for 24 hours. The solution was washed with water, a 1% sodium bicarbonate solution and evaporated. The product was purified as in Example 3 to yield 2 - (1 - decanoyloxyprop - 2 - oxy) - 2 - methyl - 4 - oxo - 1, 3 - benzodioxane identical to that in Example 3.

Example 5

1-Octanoyloxypropan-2-ol

By replacing decanoyl chloride in Example with octanoyl chloride, 1-octanoyoxypropan-1-one and subsequently 1-octanoyloxypropan-2-ol, as in Example 2, can be obtained.

Example 6

2-(1-Octanoyloxyprop-2-oxy)-2-methyl-4-oxo-1, 3-benzodioxane

By replacing 1-decanoyloxypropan-2-ol in Example 3 with 1 - octanoyloxypropan - 2 - ol, 2 - (1 - octanoyloxyprop - 2 - oxy) - 2 - methyl - 4 - oxo - 1,3 - benzodioxane can be obtained.

**Claims for the Contracting States: BE CH DE FR GB IT LI NL SE**

1. An analgesic and anti-inflammatory compound having the formula:

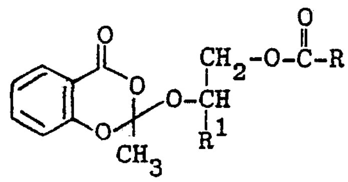

I

wherein R and $R^1$ are each an alkyl group having 1 to 15 carbon atoms, or a pharmaceutically acceptable acid addition salt thereof.

2. A compound according to Claim 1, wherein R is a straight chain alkyl group.

3. A compound according to Claim 2, wherein said straight chain alkyl group has 7 to 11 carbon atoms.

4. A compound according to any preceding claim, wherein $R^1$ is a lower alkyl group.

5. A compound according to Claim 4, wherein $R^1$ is a methyl, ethyl or propyl group.

6. 2-(1-Decanoyloxyprop-2-oxy)-2-methyl-4-oxo-1, 3-benzodioxane.

7. 2-(1-Octanoyloxyprop-2-oxy)-2-methyl-4-oxy-1, 3-benzodioxane.

8. A process for preparing an analgesic and anti-inflammatory compound, as defined in Claim 1, characterized by the step of reacting an acetyl salicylic acid derivative with a monoglycol ester having the formula:

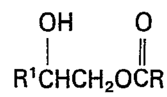

III

wherein R and $R^1$ are each alkyl radicals having from 1 to 15 carbon atoms.

9. A pharmaceutical composition, comprising a compound according to any one of Claims 1—7, and a pharmaceutically acceptable diluent or carrier therefor.

**Claims for the Contracting State: AT**

1. A method for preparing a therapeutic composition having analgesic and anti-inflammatory activity, comprising incorporating with a pharmaceutically acceptable diluent or carrier a compound having the formula I:

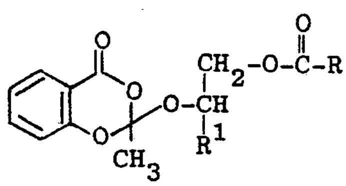

I

wherein R and $R^1$ are each an alkyl group having 1 to 15 carbon atoms, or a pharmaceutically acceptable acid addition salt thereof.

2. A method according to Claim 1, wherein R is a straight chain alkyl group.

3. A method according to Claim 2, wherein said straight chain alkyl group has 7 to 11 carbon atoms.

4. A method according to any preceding claim, wherein $R^1$ is a lower alkyl group.

5. A method according to Claim 4, wherein $R^1$ is a methyl, ethyl or propyl group.

6. A method according to Claim 1, wherein said compound of formula I is 2 - (1 - decanoyloxyprop - 2 - oxy) - 2 - methyl - 4 - oxo - 1,3 - benzodioxane.

7. A method according to Claim 1, wherein said compound of formula I is 2 - (1 - octanoyloxyprop - 2 - oxy) - 2 - methyl - 4 - oxy - 1,3 - benzodioxane.

8. A process for preparing a compound of formula I, as defined in Claim 1, characterized by the step of reacting an acetyl salicylic acid derivative with a monoglycol ester having the formula:

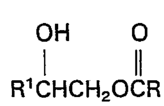

wherein R and $R^1$ are each alkyl radicals having 1 to 15 carbon atoms.

**Patentansprüche für die Vertragsstaaten: BE CH DE FR GB IT LI NL SE**

1. Eine analgetische und entzündungshemmende Verbindung der Formel

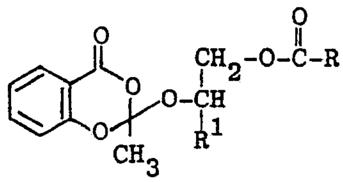

I

worin R und $R^1$ jeweils eine Alkylgruppe mit 1—15 C-Atomen bedeuten, oder ein pharmazeutisch verträgliches Säureadditionssalz derselben.

2. Eine Verbindung nach Anspruch 1, worin R für eine geradkettige Alkylgruppe steht.

3. Eine Verbindung nach Anspruch 2, worin die genannte geradkettige Alkylgruppe 7—11 C-Atome aufweist.

4. Eine Verbindung nach einem der vorhergehenden Ansprüche, worin $R^1$ eine niedere Alkylgruppe darstellt.

5. Eine Verbindung nach Anspruch 4, worin $R^1$ eine Methyl-, Äthyl- oder Propylgruppe ist.

6. 2-(1-Decanoyloxyprop-2-oxy)-2-methyl-4-oxo-1,3-benzodioxan.

7. 2-(1-Octanoyloxyprop-2-oxy)-2-methyl-4-oxo-1,3-benzodioxan.

8. Ein Verfahren zur Herstellung einer analgetischen und entzündungshemmenden Verbindung nach Anspruch 1, gekennzeichnet durch den Schritt des Umsetzens eines Acetylsalicylsäurederivates mit einem Monoglycolester der Formel

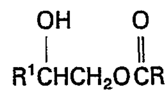

III

worin R und $R^1$ jeweils Alkylreste mit 1—15 C-Atomen darstellen.

9. Eine pharmazeutische Zusammensetzung, umfassend eine Verbindung nach einem der Ansprüche 1 bis 7, und ein pharmazeutisch verträglicher Verdünner oder Träger dafür.

**Patentansprüche für den Vertragsstaat: AT**

1. Ein Verfahren zur Herstellung einer therapeutischen Zusammensetzung mit analgetischer und entzündungshemmender Wirkung, umfassend das Einbringen einer Verbindung der Formel

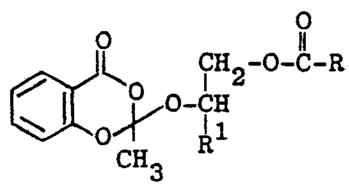

I

worin R und $R^1$ jeweils eine Alkylgruppe mit 1—15 C-Atomen bedeuten, oder eines pharmazeutisch verträglichen Säureadditionssalzes derselben, in einem pharmazeutisch verträglichen Verdünner oder Träger.

2. Ein Verfahren nach Anspruch 1, worin R eine geradkettige Alkylgruppe bedeutet.

3. Ein Verfahren nach Anspruch 2, worin die genannte geradkettige Alkylgruppe 7—11 C-Atome aufweist.

4. Ein Verfahren nach einem der vorhergehenden Ansprüche, worin $R^1$ eine niedere Alkylgruppe darstellt.

5. Ein Verfahren nach Anspruch 4, worin $R^1$ eine Methyl-, Äthyl- oder Propylgruppe ist.

6. Ein Verfahren nach Anspruch 1, worin die genannte Verbindung der Formel I 2 - (1 - Decanoyloxyprop - 2 - oxy) - 2 - methyl - 4 - oxo - 1,3 - benzodioxan ist.

7. Ein Verfahren nach Anspruch 1, worin die genannte Verbindung der Formel I 2 - (1 - Octanoyloxyprop - 2 - oxy) - 2 - methyl - 4 - oxo - 1,3 - benzodioxan ist.

8. Ein Verfahren zur Herstellung einer Verbindung der Formel I nach Anspruch 1, gekennzeichnet durch den Schritt der Umsetzung eines Acetylsalicylsäurederivatives mit einem Monoglycolester der Formel

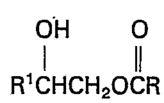

worin R und $R^1$ jeweils Alkylreste mit 1—15 C-Atomen darstellen.

**Revendications pour les Etats contractants: BE CH DE FR GB IT LI NL SE**

1. Composé analgésique et anti-inflammatoire, ayant pour formule:

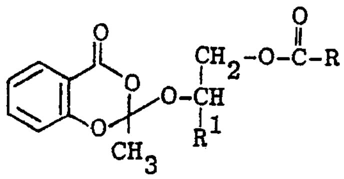

I

dans laquelle R et $R^1$ représentent chacun un groupe alkyle ayant 1 à 15 atomes de carbone, ou un de ses sels d'addition d'acide pharmaceutiquement acceptable.

2. Composé selon la revendication 1, dans lequel R est un groupe alkyle en chaîne droite.

3. Composé selon la revendication 2, dans lequel ledit groupe alkyle en chaîne droite comporte 7 à 11 atomes de carbone.

4. Composé selon l'une quelconque des revendications précédentes, dans lequel $R^1$ est un groupe alkyle inférieur.

5. Composé selon la revendication 4, dans lequel $R^1$ est un groupe méthyle, éthyle ou propyle.

6. Le 2-(1-décanoyloxyprop-2-oxy)-2-méthyl-4-oxo-1,3-benzodioxanne.

7. Le 2-(1-octanoyloxyprop-2-oxy)-2-méthyl-4-oxo-1,3-benzodioxanne.

8. Procédé pour préparer un composé analgésique et anti-inflammatoire, tel que défini à la revendication 1, caractérisé par l'étape consistant à faire réagir un dérivé de l'acide acétyl salicylique avec un ester de monoglycol ayant pour formule:

```
    OH    O
    |     ||
R¹CHCH₂OCR
```

III

dans laquelle R et $R^1$ représentent chacun des radicaux alkyles ayant de 1 à 15 atomes de carbone.

9. Composition pharmaceutique, comprenant un composé selon l'une quelconque des revendications 1 à 7, et un diluant ou véhicule pharmaceutiquement acceptable pour ce dernier.

**Revendications pour l'Etat contractant: AT**

1. Procédé pour préparer une composition thérapeutique ayant une activité analgésique et anti-inflammatoire, comprenant l'incorporation, à un diluant, véhicule ou support pharmaceutiquement acceptable, d'un composé de formule I:

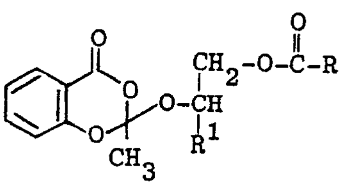

I

dans laquelle R et $R^1$ représentent chacun un groupe alkyle ayant 1 à 15 atomes de carbone, ou un de ses sels d'addition d'acide pharmaceutiquement acceptable.

2. Procédé selon la revendication 1, dans lequel R représente un groupe alkyle en chaîne droite.

3. Procédé selon la revendication 2, dans lequel ledit groupe alkyle en chaîne droite comporte 7 à 11 atomes de carbone.

4. Procédé selon l'une quelconque des revendications précédentes, dans lequel $R^1$ représente un groupe alkyle inférieur.

5. Procédé selon la revendication 4, dans lequel $R^1$ représente un groupe méthyle, éthyle ou propyle.

6. Procédé selon la revendication 1, dans lequel ledit composé de formule I est le 2 - (1 - décanoyloxyprop - 2 - oxy) - 2 - méthyl - 4 - oxo - 1,3 - benzodioxanne.

7. Procédé selon la revendication 1, dans lequel le dit composé de formule I est le 2 - (1 - octanoyloxyprop - 2 - oxy) - 2 - méthyl - 4 - oxo - 1,3 - benzodioxanne.

8. Procédé pour préparer un composé de formule I, tel que défini à la revendication 1, caractérisé par l'étape consistant à faire réagir un dérivé de l'acide acétyl salicylique avec un ester de monoglycol ayant pour formule

```
    OH    O
    |     ||
R¹CHCH₂OCR
```

dans laquelle R et $R^1$ représentent chacun des radicaux alkyles ayant 1 à 15 atomes de carbone.